(19) Europäisches Patentamt
European Patent Office
Office européen des brevets

(11) Numéro de publication : 0 446 124 A2

# (12) DEMANDE DE BREVET EUROPEEN

(21) Numéro de dépôt : 91400594.7

(51) Int. Cl.$^5$ : A61K 31/565

(22) Date de dépôt : 05.03.91

(30) Priorité : 06.03.90 FR 9002783

(43) Date de publication de la demande :
11.09.91 Bulletin 91/37

(84) Etats contractants désignés :
AT BE CH DE DK FR GB GR IT LI LU NL SE

(71) Demandeur : ROUSSEL-UCLAF
35, boulevard des Invalides
F-75007 Paris (FR)

(72) Inventeur : Grandadam, Jean André
56, Avenue Gabriel Péri
F-94100 Saint maur des Fosses (FR)

(74) Mandataire : Tonnellier, Marie-José et al
111, route de Noisy B.P. no 9
F-93230 Romainville (FR)

## (54) Utilisation de composés antiprogestomimétiques pour synchroniser la mise bas chez les animaux d'élevage.

(57) La présente invention a pour objet l'utilisation de composés anti-progestomimétiques pour la fabrication de compositions destinées à la synchronisation de la mise bas chez les animaux d'élevage.

EP 0 446 124 A2

Jouve, 18, rue Saint-Denis, 75001 PARIS

# NOUVELLE UTILISATION DE COMPOSES ANTI-PROGESTOMIMETIQUES CHEZ LES ANIMAUX D'ELEVAGE

La présente invention concerne une nouvelle utilisation de composés antiprogestomimétiques chez les animaux d'élevage.

L'invention a pour objet l'utilisation de composés anti-progestomimétiques pour la fabrication de compositions destinées à la synchronisation de la mise bas chez les animaux d'élevage.

L'invention a plus particulièrement pour objet l'utilisation, caractérisée en ce que les composés doués d'activité anti-progestomimétique répondent à la formule générale (I) :

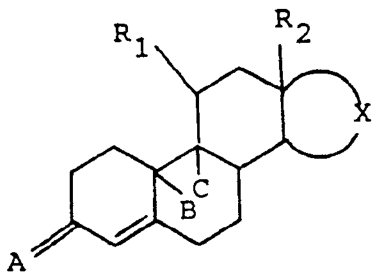

(I)

dans laquelle $R_1$ représente un radical hydrocarboné renfermant de 1 à 18 atomes de carbone et éventuellement un ou plusieurs hétéroatomes identiques ou différents, lié au noyau stéroïde par un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

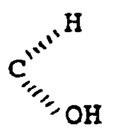 , 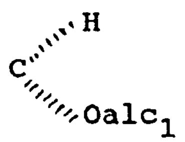 , 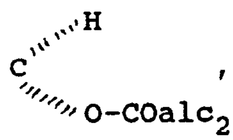 ,

un groupement C=NOH, un groupement C=NOalc$_3$ ou un groupement $CH_2$, alc$_1$, alc$_2$ et alc$_3$ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

L'invention a notamment pour objet l'utilisation, caractérisée en ce que les composés doués d'activité anti-progestomimétique répondent à la formule générale (I) dans laquelle $R_1$ représente un radical hydrocarboné renfermant de 1 à 18 atomes de carbone contenant au moins un atome d'azote, de phosphore ou de silicium lié au noyau stéroïde par un atome de carbone.

$R_2$ représente de préférence un radical alkyle saturé, linéaire ou ramifié, renfermant de 1 à 4 atomes de carbone, par exemple un radical méthyle, éthyle, n-propyle ou butyle.

Lorsque alc$_1$, alc$_2$ ou alc$_3$ représente un radical alkyle, il s'agit de préférence du radical méthyle, éthyle, n-propyle ou isopropyle.

Lorsque alc$_1$, alc$_2$ ou alc$_3$ représente un radical aralkyle, il s'agit de préférence du radical benzyle.

X représente de préférence le reste d'un cycle pentagonal éventuellement substitué.

Les composés de formule (I) sont des composés connus décrits et revendiqués dans le brevet européen 0 057 115 et les brevets français 2 566 779 et 2 625 505 où ils sont présentés comme doués de propriétés différentes, propriétés pharmacologiques et notamment une activité anti-progestomimétique.

Certains produits de formule (I) décrits et revendiqués dans la demande de brevet français 89 10648 déposée le 8 août 1989 ne sont pas encore publiés ; leur préparation est donnée ci-après dans la partie expérimentale.

On vient de découvrir que les composés de formule (I) permettaient une remarquable synchronisation de la mise bas chez les animaux d'élevage comme le montrent les résultats exprimés ci-après dans la partie expérimentale.

Après l'injection de produit à une dose convenable, les produits de formule (I)entraînent la mise bas dans un laps de temps inférieur à 36 heures voire inférieur à 24 heures.

Parmi les utilisations préférées, on peut citer l'utilisation caractérisée en ce que dans le produit de formule (I), B et C forment ensemble une double liaison, celle où dans le produit de formule (I), $R_2$ représente un radical méthyle, celle où dans le produit de formule (I), le groupement C=A représente un groupement

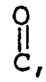

celle où dans le produit de formule (I), $R_1$ représente un radical :

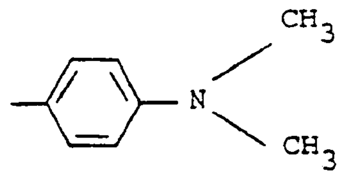

celle où dans le produit de formule (I), X représente le reste d'un cycle :

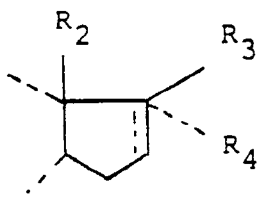

dans lequel $R_2$ conserve la même signification que précédemment, $R_3$ et $R_4$, identiques ou différents, représentent soit un atome d'hydrogène, soit un radical OH, $Oalc_4$, $O\text{-}COalc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

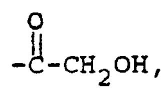

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO\text{-}CO_2H$, ou $CO\text{-}CO_2alc_7$ dans lequel $alc_7$ représente un radical alkyle renfermant de 1 à 8 atomes de carbone, soit un radical

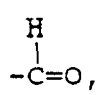

soit un radical

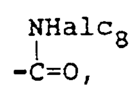

dans lequel $alc_8$ représente un radical alkyle renfermant de 1 à 8 atomes de carbone ou un radical aralkyle renfermant de 7 à 15 atomes de carbone, soit un radical $-C\equiv N$, soit $R_3$ et $R_4$ forment ensemble un radical :

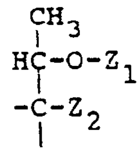

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone et notamment celle où X représente le reste d'un cycle :

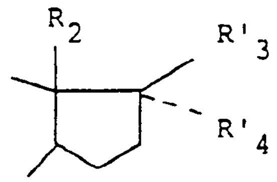

dans lequel $R_2$ conserve la même signification que précédemment et $R'_3$ représente un radical OH, et $R'_4$ un radical alkynyle ou alkényle renfermant jusqu'à 4 atomes de carbone.

Parmi les formes préférées de l'invention, on peut citer l'utilisation où le composé anti-progestomimétique est le 17béta-hydroxy 11béta-(4-diméthylaminophényl) 17alpha-(prop-1-ynyl) estra-4,9-dièn-3-one (appelé ci-après produit A) ou encore celle où le composé anti-progestomimétique est le (Z) 11béta-[4-(diméthylamino) phényl] 17béta-hydroxy 17alpha-(1-propényl) estra-4,9-dièn-3-one ou encore le butanedioate de 11béta-[4-(méthylthio) phényl] 3-oxo 17alpha-(1-propynyl) estra-4,9-dièn-17béta-yle ou l'un de ses sels alcalins et notamment son sel de sodium ou encore le butanedioate de 11béta-[4-(diméthylamino) phényl] 3-oxo 17alpha-(1-propynyl) estra-4,9-dièn-17béta-yle ou l'un de ses sels alcalins et notamment son sel de sodium.

La préparation des deux derniers produits mentionnés est indiqué ci-après dans la partie expérimentale.

L'invention a plus particulièrement pour objet l'utilisation, caractérisée en ce que l'administration a lieu chez la truie ou la vache ou encore la brebis, la chèvre, la jument.

Les compositions sont administrées de préférence par voie injectable.

Dans un mode d'utilisation préférée, on administre par voie injectable chez la truie entre le 109ème et le 114ème jour de la gestation le 17bèta-hydroxy 11béta-(4-diméthylaminophényl) 17alpha-(prop-1-ynyl) estra-4,9-dièn-3-one à une dose comprise entre 1 mg et 3 mg par kg de poids d'animal, par exemple 2 mg/kg de poids d'animal.

Les exemples suivants illustrent l'invention sans toutefois la limiter.

**<u>PREPARATION 1</u> : Butanedioate de 11béta-[4-(diméthylamino) phényl] 3-oxo 17alpha-(1-propynyl) estra-4,9-dièn-17béta-yle et de sodium.**

**<u>STADE A</u> :** Succinate acide 11béta-[4-(diméthylamino) phényl] 3-oxo 17alpha-(1-propynyl) estra-4,9-dièn-17béta-yle.

Le milieu réactionnel est préparé en ajoutant 2,15 g d'anhydride succinique, 2,2 $cm^3$ de triéthylamine et 215 mg de 4-(diméthylamino) pyridine à une solution de 2,15 g de 11béta-[4-(diméthylamino) phényl] 17béta-hydroxy 17alpha-(1-propynyl) estra-4,9-dièn-3-one dans 22 $cm^3$ de chloroforme, puis chauffé au reflux pendant 42 heures et 430 mg de 4-(diméthylamino) pyridine et 4,4 $cm^3$ de triéthylamine sont rajoutés. Le reflux est poursuivi pendant 26 heures et la solution est alors versée sur un mélange eau-glace. Après décantation de la phase organique, celle-ci est lavée puis séchée et le chloroforme est distillé pour conduire à un extrait sec de couleur brune. La phase aqueuse est acidifiée avec de l'acide chlorhydrique 0,5N puis neutralisée par addition d'acétate de sodium. On extrait de nouveau à l'acétate d'éthyle et la nouvelle phase organique est lavée à l'eau, séchée et après distillation du solvant conduit à un résidu que l'on réunit avec le précédent. Le produit est purifié sur colonne de silice en éluant avec un mélange éther-acétate d'éthyle (9-1) à 3% d'acide acétique et recristallisé deux fois dans un mélange éther-chlorure de méthylène. On obtient 1,435 g du produit recherché. F ≈ 165°C. $[alpha]_D$ = +97° (c = 0,8% dans $CHCl_3$).

Rf ≈ 0,40 (chromatographie sur couche mince, support : $SiO_2$, éluant : éther 9 - acétate d'éthyle 1 - acide acétique 3%).

**STADE B** : Butanedioate de sodium et de 11béta-[4-(diméthylamino) phényl] 3-oxo 17alpha-(1-propynyl) estra-4,9-dièn-17béta-yle.

Dans un ballon muni d'une agitation magnétique, on introduit 3 g du produit préparé au stade A ci-dessus et 94 $cm^3$ d'éthanol et verse ensuite une solution de 433 mg de bicarbonate de sodium dans 94 $cm^3$ d'eau. Après 30 minutes d'agitation à température ambiante, l'éthanol est passé par azéotropie et la solution restante est filtrée sur membrane millipore [R] (0,45 microns) et lyophilisée. On obtient 2,88 g du produit recherché.
$[alpha]_D$ = +48,5 ± 1,5° (c = 1% dans l'eau).
Rf = 0,54 (chromatographie sur couche mince, support : KC 18 Whatman [R] , éluant : méthanol-solution aqueuse d'acétate d'ammonium 0,05 molaire (80-20).

**PREPARATION 2 : Butanedioate de sodium et de 11béta-[4-(méthylthio) phényl] 3-oxo 17alpha-(1-propynyl) estra-4,9-dièn-17béta-yle.**

**STADE A** : Butanedioate acide de 11béta-[4-(méthylthio) phényl] 3-oxo 17alpha-(1-propynyl) estra-4,9-dièn-17béta-yle.

Dans un ballon muni d'une agitation magnétique et d'un réfrigérant, 1,5 g de 17béta-hydroxy 11béta-[4-(méthylthio) phényl] 17alpha-(1-propynyl) estra-4,9-dièn-3-one, 15,3 $cm^3$ de chloroforme sont mélangés puis 1,86 g d'anhydride succinique, 6 $cm^3$ de triéthylamine et 794 mg de 4-(diméthylamino) pyridine sont ajoutés et le tout est chauffé au reflux pendant 94 heures, versé dans de l'acide chlorhydrique 1N et extrait au chloroforme. La phase chloroformique est lavée à l'eau, séchée sur sulfate de sodium et le solvant est éliminé sous pression réduite à 40°C. On obtient 2,26 g de produit brut que l'on chromatographie sur une colonne de silice Kieselgel 60H [R] (éluant : (chlorure de méthylène 97,5-méthanol 2,5)-acide acétique 1%). Après recristallisation dans le mélange chlorure de méthylène-éther isopropylique, il se forme 826 mg de cristaux du produit recherché. F = 158°C.
Rf = 0,61 (chromatographie sur couche mince, support : KC 18 Whatman [R] , éluant : méthanol-solution aqueuse d'acétate d'ammonium 0,05 molaire (70-30).

**STADE B** : Butanedioate de sodium et de 11béta-[4-(méthylthio) phényl] 3-oxo 17alpha-(1-propynyl) estra-4,9-dièn-17béta-yle.

En opérant de la même manière qu'au stade B de la préparation 1 avec 108 mg de bicarbonate de sodium dans 21,5 $cm^3$ d'eau et 719 mg du produit préparé au stade A dans 21,5 $cm^3$ d'éthanol, on obtient 720 mg d'un lyophilisat correspondant au produit recherché.
$[alpha]_D$ = +74,5 ± 1,5° (c = 1% dans l'eau).
Rf = 0,61 (chromatographie sur couche mince, support : KC 18 Whatman [R] , éluant : méthanol-solution aqueuse d'acétate d'ammonium 0,05 molaire (70-30).

**EXEMPLES DE COMPOSITION PHARMACEUTIQUES.**

a) On a préparé des solutions injectables renfermant 150 mg de 17béta-hydroxy 11béta-(4-diméthylaminophényl) 17alpha-(prop-1-ynyl) estra-4,9-dièn-3-one (produit A).
b) On a préparé des solutions injectables renfermant 300 mg de produit A.
c) On a préparé des solutions injectables renfermant 600 mg de produit A.
d) On a préparé des solutions injectables renfermant 800 mg de produit A.

**ESSAI BIOLOGIQUE**

L'essai a été réalisé chez des truies. Les truies ont été sélectionnées en fonction des critères suivants :
- truies de même origine (même schéma de sélection),
- truies de même rang de parturition (3ème),
- saillie par le même verrat ou insémination artificielle avec semence de même origine,
- nombre de saillies ou d'inséminations identiques,
- saillie ou insémination sur première chaleur post-sevrage (intervalle sevrage saillie identique ou très proche),
- durée d'allaitement identique.

Les truies sont divisées en 3 lots de n animaux :

– un lot témoin ne reçoit aucun produit,

– un lot reçoit une injection intra-musculaire de 2 mg/kg de produit A ou 17béta-hydroxy 11béta-(4-diméthylaminophényl) 17alpha-(prop-1-ynyl) estra-4,9-dièn-3-one, le 113ème jour de la gestation,

– un lot reçoit une injection intra-musculaire de 175 mcg de cloprosténol sous forme de PLANATE R par truie, le 113 ème jour de la gestation.

On a mesuré l'intervalle de temps entre l'injection du principe actif et la mise bas : le tableau suivant résume les résultats obtenus.

1.- Animaux traités (Ho = 9 heures le 113ème jour de gestation.

| Produits essayés | - 20 H | 20 à 30 H | 30 à 36 H | + 36 H |
|---|---|---|---|---|
| Produit A<br>n = 24 | 4%<br>1 | 92%<br>22 | 4%<br>1 | |
| Cloprosténol<br>n = 23 | 4,5%<br>1 | 78%<br>18 | 4,5%<br>1 | 13%<br>3 |

2.- Animaux témoins : essais simultanés

| | 112J | 113J | 114J | 115J | 116J | 117J | 118J | |
|---|---|---|---|---|---|---|---|---|
| TEMOINS<br>n = 23 | 9%<br>2 | 13%<br>3 | 35%<br>8 | 17%<br>4 | 17%<br>4 | 4,5%<br>1 | 4,5%<br>1 | |

**Conclusion :**

L'administration du produit A entraîne une synchronisation de la mise bas très intéressante : 92% des animaux traités mettent bas de 20 à 30 heures après l'administration du produit.

**Revendications**

**1.-** Utilisation de composés anti-progestomimétiques pour la fabrication de compositions destinées à la synchronisation de la mise bas chez les animaux d'élevage.

**2.-** Utilisation selon la revendication 1, caractérisée en ce que les composés doués d'activité anti-progestomimétique répondent à la formule générale (I) :

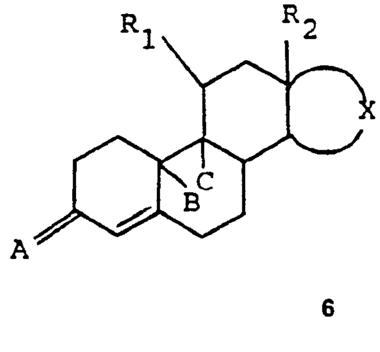

(I)

dans laquelle $R_1$ représente un radical hydrocarboné renfermant de 1 à 18 atomes de carbone et éventuellement un ou plusieurs hétéroatomes identiques ou différents, lié au noyau stéroïde par un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

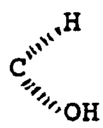 , 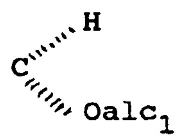 , 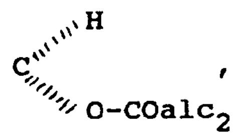 ,

un groupement C=NOH, un groupement $C=NOalc_3$ ou un groupement $CH_2$, $alc_1$, $alc_2$ et $alc_3$ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

**3.-** Utilisation selon la revendication 2, caractérisée en ce que les composés doués d'activité anti-progestomimétique répondent à la formule générale (I) dans laquelle $R_1$ représente un radical hydrocarboné renfermant de 1 à 18 atomes de carbone contenant au moins un atome d'azote, de phosphore ou de silicium lié au noyau stéroïde par un atome de carbone.

**4.-** Utilisation selon la revendication 2 ou 3, caractérisée en ce que dans le produit de formule (I), B et C forment ensemble une double liaison.

**5.-** Utilisation selon l'une quelconque des revendications 2 à 4, caractérisée en ce que dans le produit de formule (I), $R_2$ représente un radical méthyle.

**6.-** Utilisation selon l'une quelconque des revendications 2 à 5, caractérisée en ce que dans le produit de formule (I), le groupement C=A représente un groupement

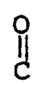.

**7.-** Utilisation selon l'une quelconque des revendications 2 à 6, caractérisée en ce que dans le produit de formule (I), $R_1$ représente un radical :

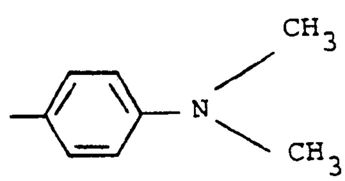

**8.-** Utilisation selon l'une quelconque des revendications 2 à 7, caractérisée en ce que dans le produit de formule (I), X représente le reste d'un cycle :

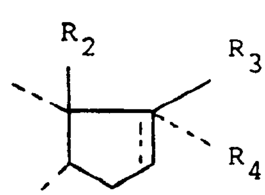

dans lequel $R_2$ conserve la même signification que précédemment, $R_3$ et $R_4$, identiques ou différents, représentent soit un atome d'hydrogène, soit un radical OH, $Oalc_4$, $O-COalc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

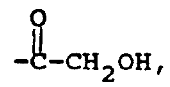

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO-CO_2H$, ou $CO-CO_2alc_7$ dans lequel $alc_7$ représente un radical alkyle renfermant de 1 à 8 atomes de carbone, soit un radical

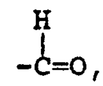

soit un radical

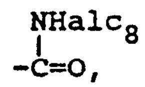

dans lequel $alc_8$ représente un radical alkyle renfermant de 1 à 8 atomes de carbone ou un radical aralkyle renfermant de 7 à 15 atomes de carbone, soit un radical -C≡N, soit $R_3$ et $R_4$ forment ensemble un radical :

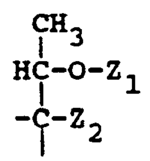

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone.

**9.-** Utilisation selon la revendication 8, caractérisée en ce que dans le produit de formule (I), X représente le reste d'un cycle :

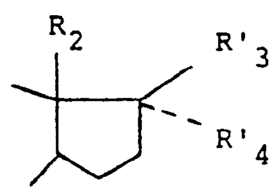

dans lequel $R_2$ conserve la même signification que précédemment et $R'_3$ représente un radical OH, et $R'_4$ un radical alkynyle ou alkényle renfermant jusqu'à 4 atomes de carbone.

**10.-** Utilisation selon la revendication 9, caractérisée en ce que le composé anti-progestomimétique est le 17béta-hydroxy 11béta-(4-diméthylaminophényl) 17alpha-(prop-1-ynyl) estra-4,9-dièn-3-one.

**11.-** Utilisation selon la revendication 9, caractérisée en ce que le composé anti-progestomimétique est le (Z) 11béta-[4-(diméthylamino) phényl] 17béta-hydroxy 17alpha-(1-propényl) estia-4,9-dièn-3-one.

**12.-** Utilisation selon la revendication 9, caractérisée en ce que le composé anti-progestomimétique est le butanedioate de 11béta-[4-(méthylthio) phényl] 3-oxo 17alpha-(1-propynyl) estra-4,9-dièn-17béta-yle ou l'un de ses sels alcalins et notamment son sel de sodium.

**13.-** Utilisation selon la revendication 9, caractèrisée en ce que le composé anti-progestomimétique est le butanedioate de 11béta-[4-(diméthylamino) phényl] 3-oxo 17alpha-(1-propynyl) estra-4,9-dièn-17béta-yle ou l'un de ses sels alcalins et notamment son sel de sodium.

**14.-** Utilisation selon l'une quelconque des revendications 1 à 13, caractérisée en ce que l'administration a lieu chez la truie.

**15.-** Utilisation selon l'une quelconque des revendications 1 à 13, caractérisée en ce que l'administration a lieu chez la vache.

**16.-** Utilisation selon l'une quelconque des revendications 1 à 15, caractérisée en ce que l'administration du principe actif a lieu par voie injectable.

**17.-** Utilisation selon l'une quelconque des revendications 1 à 14, caractérisée en ce que l'on administre par voie injectable chez la truie entre le 109ème et le 114ème jour de la gestation le produit de la revendication 10 à une dose comprise entre 1 mg et 3 mg par kg de poids d'animal.

**18.-** Utilisation selon la revendications 17, caractérisée en ce que l'on administre chez la truie par injection une dose de 2 mg/kg de poids d'animal du produit de la revendication 10.

**Revendications pour l'Etat contractant suivant : GR**

**1.-** Utilisation de composés anti-progestomimétiques pour la fabrication de compositions destinées à la synchronisation de la mise bas chez les animaux d'élevage.

**2.-** Utilisation selon la revendication 1, caractérisée en ce que les composés doués d'activité anti-progestomimétique répondent à la formule générale (I) :

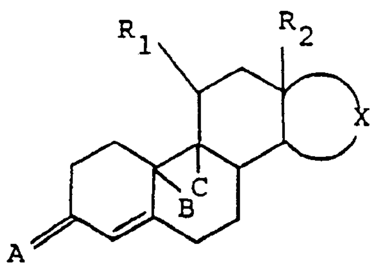

(I)

dans laquelle $R_1$ représente un radical hydrocarboné renfermant de 1 à 18 atomes de carbone et éventuellement un ou plusieurs hétéroatomes identiques ou différents, lié au noyau stéroïde par un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

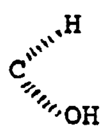 , 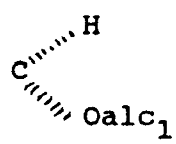 , 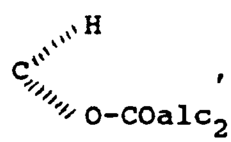 ,

un groupement C=NOH, un groupement C=NOalc$_3$ ou un groupement $CH_2$, alc$_1$, alc$_2$ et alc$_3$ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

**3.-** Utilisation selon la revendication 2, caractérisée en ce que les composés doués d'activité anti-progestomimétique répondent à la formule générale (I) dans laquelle $R_1$ représente un radical hydrocarboné renfermant de 1 à 18 atomes de carbone contenant au moins un atome d'azote, de phosphore ou de silicium lié au noyau stéroïde par un atome de carbone.

**4.-** Utilisation selon la revendication 2 ou 3, caractérisée en ce que dans le produit de formule (I), B et C forment ensemble une double liaison.

**5.-** Utilisation selon l'une quelconque des revendications 2 à 4, caractérisée en ce que dans le produit de formule (I), $R_2$ représente un radical méthyle.

**6.-** Utilisation selon l'une quelconque des revendications 2 à 5, caractérisée en ce que dans le produit de formule (I), le groupement C=A représente un groupement

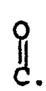

**7.-** Utilisation selon l'une quelconque des revendications 2 à 6, caractérisée en ce que dans le produit de formule (I), $R_1$ représente un radical :

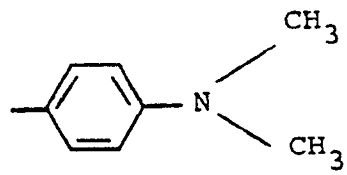

**8.-** Utilisation selon l'une quelconque des revendications 2 à 7, caractérisée en ce que dans le produit de formule (I), X représente le reste d'un cycle :

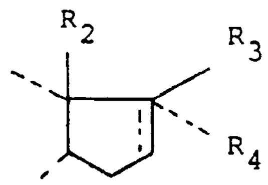

dans lequel $R_2$ conserve la même signification que précédemment, $R_3$ et $R_4$, identiques ou différents, représentent soit un atome d'hydrogène, soit un radical OH, $Oalc_4$, $O\text{-}COalc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

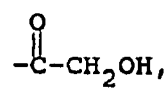

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO\text{-}CO_2H$, ou $CO\text{-}CO_2alc_7$ dans lequel $alc_7$ représente un radical alkyle renfermant de 1 à 8 atomes de carbone, soit un radical

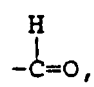

soit un radical

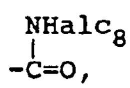

dans lequel $alc_8$ représente un radical alkyle renfermant de 1 à 8 atomes de carbone ou un radical aralkyle renfermant de 7 à 15 atomes de carbone, soit un radical -C≡N, soit $R_3$ et $R_4$ forment ensemble un radical :

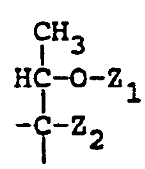

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone.

**9.-** Utilisation selon la revendication 8, caractérisée en ce que dans le produit de formule (I), X représente le reste d'un cycle :

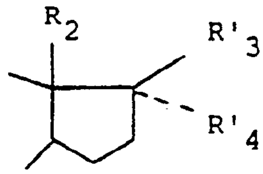

dans lequel $R_2$ conserve la même signification que précédemment et $R'_3$ représente un radical OH, et $R'_4$ un radical alkynyle ou alkényle renfermant jusqu'à 4 atomes de carbone.

**10.-** Utilisation selon la revendication 9, caractérisée en ce que le composé anti-progestomimétique est le 17béta-hydroxy 11béta-(4-diméthylaminophényl) 17alpha-(prop-1-ynyl) estra-4,9-dièn-3-one.

**11.-** Utilisation selon la revendication 9, caractérisée en ce que le composé anti-progestomimétique est le (Z) 11béta-[4-(diméthylamino) phényl] 17béta-hydroxy 17alpha-(1-propényl) estra-4,9-dièn-3-one.

**12.-** Utilisation selon la revendication 9, caractérisée en ce que le composé anti-progestomimétique est le butanedioate de 11béta-[4-(méthylthio) phényl] 3-oxo 17alpha-(1-propynyl) estra-4,9-dièn-17béta-yle ou l'un de ses sels alcalins et notamment son sel de sodium.

**13.-** Utilisation selon la revendication 9, caractérisée en ce que le composé anti-progestomimétique est le butanedioate de 11béta-[4-(diméthylamino) phényl] 3-oxo 17alpha-(1-propynyl) estra-4,9-dièn-17béta-yle ou l'un de ses sels alcalins et notamment son sel de sodium.

**14.-** Utilisation selon l'une quelconque des revendications 1 à 13, caractérisée en ce que l'administration a lieu chez la truie.

**15.-** Utilisation selon l'une quelconque des revendications 1 à 13, caractérisée en ce que l'administration a lieu chez la vache.

**16.-** Utilisation selon l'une quelconque des revendications 1 à 15, caractérisée en ce que l'administration du principe actif a lieu par voie injectable.

**17.-** Utilisation selon l'une quelconque des revendications 1 à 14, caractérisée en ce que l'on administre par voie injectable chez la truie entre le 109ème et le 114ème jour de la gestation le produit de la revendication 10 à une dose comprise entre 1 mg et 3 mg par kg de poids d'animal.

**18.-** Utilisation selon la revendications 17, caractérisée en ce que l'on administre chez la truie par injection une dose de 2 mg/kg de poids d'animal du produit de la revendication 10.

**19.-** Procédé de préparation de compositions destinées à la synchronisation de la mise-bas chez les animaux d'élevage, caractérisé en ce que l'on met à titre de principe actif au moins un composé anti-progestomimétique sous une forme destinée à cet usage.

**20.-** Procédé de préparation de compositions destinées à la synchronisation de la mise-bas chez les animaux d'élevage, caractérisé en ce que l'on met à titre de principe actif au moins un des composés doués d'activité anti-progestomimétique répondant à la formule générale (I) :

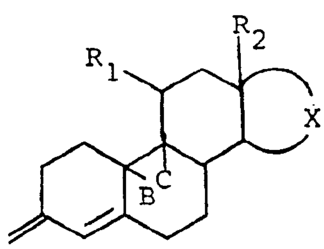

(I)

dans laquelle $R_1$ représente un radical hydrocarboné renfermant de 1 à 18 atomes de carbone et éventuellement un ou plusieurs hétéroatomes identiques ou différents, lié au noyau stéroïde par un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

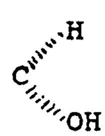 , 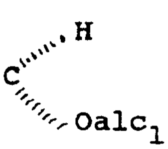 , 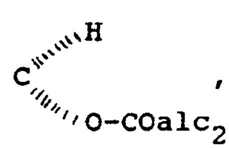 ,

un groupement C=NOH, un groupement C=NOalc$_3$ ou un groupement $CH_2$, alc$_1$, alc$_2$ et alc$_3$ représentant un radical alkyle renfermant de 1 à 8 atomes de carbone ou un groupement aralkyle renfermant de 7 à 15 atomes de carbone et B et C forment ensemble une double liaison ou un pont époxyde, ou de leurs sels d'addition avec les acides.

**21.-** Procédé selon la revendication 20, caractérisé en ce que l'on met à titre de principe actif au moins un des composés répondant à la formule (I) dans laquelle $R_1$ représente un radical hydrocarboné renfermant de 1 à 18 atomes de carbone contenant au moins un atome d'azote, de phosphore ou de silicium lié au noyau stéroïde par un atome de carbone.

**22.-** Procédé selon la revendication 20 ou 21, caractérisé en ce que l'on met à titre de principe actif au moins un des composés répondant à la formule (I) dans laquelle B et C forment ensemble une double liaison.

**23.-** Procédé selon l'une quelconque des revendications 20 à 22, caractérisé en ce que l'on met à titre de principe actif au moins un des composés répondant à la formule (I) dans laquelle $R_2$ représente un radical méthyle.

**24.-** Procédé selon l'une quelconque des revendications 20 à 23, caractérisé en ce que l'on met à titre de principe actif au moins un des composés répondant à la formule (I) dans laquelle legroupement C=A représente un groupement

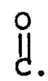

**25.-** Procédé selon l'une quelconque des revendications 20 à 24, caractérisé en ce que l'on met à titre de principe actif au moins un des composés répondant à la formule (I) dans laquelle $R_1$ représente un radical :

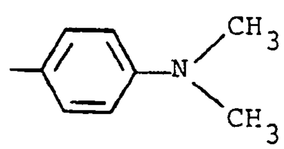

**26.-** Procédé selon l'une quelconque des revendications 20 à 25, caractérisé en ce que l'on met à titre de principe actif au moins un des composés répondant à la formule (I) dans laquelle X représente le reste d'un cycle :

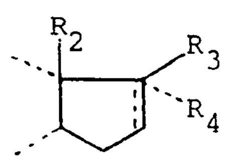

dans lequel $R_2$ conserve la même signification que précédemment, $R_3$ et $R_4$, identiques ou différents, représentent soit un atome d'hydrogène, soit un radical OH, Oalc$_4$, O-COalc$_5$, alc$_4$ et alc$_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

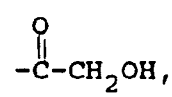

soit un radical $-COCH_2OCO$alc$_6$, dans lequel alc$_6$ représente un radical alkyle renfermant de 1 à 8 atomes de carbone éventuellement substitué ou un radical aralkyle renfermant de 7 à 15 atomes de carbone, soit un radical CO-$CO_2$H, ou CO-$CO_2$alc$_7$ dans lequel alc$_7$ représente un radical alkyle renfermant de 1 à 8 atomes de carbone, soit un radical

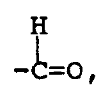

soit un radical

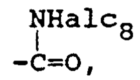

dans lequel $alc_8$ représente un radical alkyle renfermant de 1 à 8 atomes de carbone ou un radical aralkyle renfermant de 7 à 15 atomes de carbone, soit un radical -C≡N, soit $R_3$ et $R_4$ forment ensemble un radical :

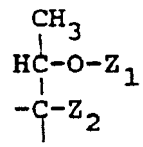

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone.

**27.-** Procédé selon la revendication 26, caractérisé en ce que l'on met à titre de principe actif des composés répondant à la formule (I) dans laquelle X représente le reste d'un cycle :

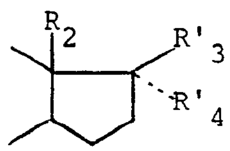

dans lequel $R_2$ conserve la même signification que précédemment et $R'_3$ représente un radical OH, et $R'_4$ un radical alkynyle ou alkényle renfermant jusqu'à 4 atomes de carbone.

**28.-** Procédé selon la revendication 19 ou 20, caractérisé en ce que le composé anti-progestomimétique est le 17béta-hydroxy 11béta-(4-diméthylaminophényl) 17alpha-(prop-1-ynyl) estra-4,9-dièn-3-one.

**29.-** Procédé selon la revendication 19 ou 20, caractérisé en ce que le composé anti-progestomimétique est le (Z) 11béta-[4-(diméthylamino) phényl] 17béta-hydroxy 17alpha-(1-propényl) estra-4,9-dièn-3-one.

**30.-** Procédé selon la revendication 19 ou 20, caractérisé en ce que le composé anti-progestomimétique est le butanedioate de 11béta-[4-(méthylthio) phényl] 3-oxo 17alpha-(1-propynyl) estra-4,9-dièn-17béta-yle ou l'un de ses sels alcalins et notamment son sel de sodium.

**31.-** Procédé selon la revendication 19 ou 20, caractérisé en ce que le composé anti-progestomimétique est le butanedioate de 11béta-(4-(diméthylamino) phényl] 3-oxo 17alpha-(1-propynyl) estra-4,9-dièn-17béta-yle ou l'un de ses sels alcalins et notamment son sel de sodium.

**32.-** Procédé de préparation de compositions destinées à la synchronisation de la mise-bas chez les animaux d'élevage, caractérisé en ce que l'on met le composé anti-progestomimétique sous forme injectable.